# EUROPEAN PATENT APPLICATION

(11) **EP 4 850 674 A1**
(43) Date of publication of application: **09.12.2026**
(21) Application number: 25747994.9
(22) Date of filing: 27.01.2025
(51) Int. Cl.: A61K 31/519, A61K 31/498, A61P 35/00, A61P 11/00

(54) **ORAL PHARMACEUTICAL COMPOSITION OF PDE4B INHIBITOR COMPOUND AND PREPARATION METHOD THEREFOR**

(30) Priority: 04.02.2024 CN 202410159158
(71) Applicant: Xizang Haisco Pharmaceutical Co., Ltd., Shannan, Xizang 856099 (CN)
(72) Inventor: WANG, Li, Chengdu, Sichuan 611130 (CN); WANG, Jianwei, Chengdu, Sichuan 611130 (CN)
(74) Representative: AWA Denmark A/S
(86) International application number: PCT/CN2025/075393
(87) International publication number: WO 2025/162431

(57) **Abstract**

Provided are an oral pharmaceutical composition containing a PDE4B inhibitor compound as shown in formula (I) and a preparation method therefor. The composition contains the compound of formula (I), a filler, an adhesive, a disintegrant, a solubilizer, and a lubricant. The composition can be prepared into oral preparation forms such as a tablet, a capsule, a granule, and a dispersible tablet, and has the advantages of a high bioavailability, good content uniformity, good stability, easy industrialization, etc.

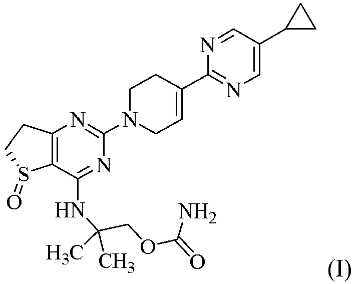

## Description

### Technical Field

The present invention relates to the technical field of chemical pharmaceuticals, and in particular to an oral pharmaceutical composition of a PDE4B inhibitor compound and a preparation method therefor.

### Background Art

Idiopathic pulmonary fibrosis (IPF) is a chronic, progressive, fibrosing interstitial lung disease confined to the lungs and predominantly affecting middle-aged and elderly populations. Its characteristic manifestations on pulmonary histology and/or chest high-resolution CT (HRCT) are consistent with usual interstitial pneumonia (UIP) and its etiology is unclear. Based on the disease course, the disease can be classified into acute, subacute and chronic types. Most cases occur sporadically. Statistically, the annual prevalence in the general population ranges from about 2 to 29 per 100,000 persons, presenting a gradual rising trend, with an estimated annual growth rate of 11%. There are approximately 100,000 patients with idiopathic pulmonary fibrosis in the United States, and approximately 110,000 in the European Union, with 35,000 new IPF cases diagnosed in the European Union each year. In Japan, the annual prevalence of IPF in the general population is about 2.23 to 10 per 100,000 persons, while the actual figure is far higher than this range. As a country facing severe population aging, China has seen a year-on-year increase in the number of IPF patients, with a conservative estimate of at least around 500,000 cases. As a chronic interstitial lung disease, IPF has an insidious onset and a progressively worsening disease course, and may also present with acute exacerbations. The median survival after IPF diagnosis is only 2.8 years. Its mortality rate exceeds that of most malignancies, earning IPF the moniker of a "tumour-like disease".

Current therapeutic options are limited. Conventionally, the therapies involve glucocorticoids alone or in combination with cytotoxic drugs, and their dosage and treatment course are determined according to the patient's specific condition. The currently recommended treatment regimen involves glucocorticoids in combination with cyclophosphamide or azathioprine. Other therapeutic drugs include N-acetylcysteine, γ-interferon and pirfenidone (TNF-α inhibitor), colchicine, penicillamine, etc. The clinical efficacy of these drugs remains to be further validated. This technical solution provides an oral pharmaceutical composition of a phosphodiesterase (PDE) inhibitor compound and a preparation method therefor, which composition is intended for the treatment of idiopathic pulmonary fibrosis (IPF) and other related diseases.

Phosphodiesterases (PDEs) are a family of hydrolases that hydrolyse cyclic adenosine monophosphate (cAMP) and cyclic guanosine monophosphate (cGMP), and serve as an important node regulating the levels of cAMP and cGMP. PDEs are classified into three categories: phosphodiesterases specifically hydrolysing cAMP, including PDE4, PDE7 and PDE8; phosphodiesterases specifically hydrolysing cGMP, including PDE5, PDE6 and PDE9; phosphodiesterases capable of hydrolysing both cAMP and cGMP, including PDE1, PDE2, PDE3, PDE10 and PDE11.

PDE4 enzymes are widely expressed in various tissues. They are generally divided into four subtypes: PDE4A, PDE4B, PDE4C and PDE4D, and have the functions including regulation of brain function, monocyte and macrophage function, and vascular smooth muscle cell proliferation. Although PDE4 is expressed in most cells, the catalytic hydrolysis of cAMP in immune cells is primarily dependent on PDE4. Therefore, PDE4 inhibitors may be used in the treatment of a variety of inflammatory diseases, including psoriasis, neuroinflammation, chronic obstructive pulmonary disease (COPD), asthma, ankylosing spondylitis (AS), inflammatory bowel disease (IBD), atopic dermatitis, rheumatoid arthritis (RA), systemic lupus erythematosus (SLE), etc.

The present invention relates to the field of pharmaceutical preparations, and in particular to an oral pharmaceutical composition of a small molecule phosphodiesterase (PDE) inhibitor compound (I). The present invention further relates to a method for preparing the pharmaceutical composition, and the use of the pharmaceutical composition in a drug for treating diseases or conditions associated with phosphodiesterase (PDE) receptors. Specific solubilization techniques, compositions and preparation methods are used in the preparation technology to obtain a preparation with a high bioavailability, good content uniformity and good stability, which is suitable for industrial-scale production. The preparation is intended for the treatment of idiopathic pulmonary fibrosis and other related diseases, thereby enriching the existing therapeutic options available for such diseases.

### Summary of the Invention

The present invention provides an oral pharmaceutical composition, comprising:
an active ingredient, wherein the active ingredient is a compound of formula (I) or a pharmaceutically acceptable salt thereof, 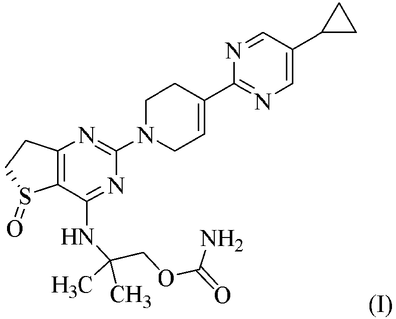
wherein the active ingredient is present in the pharmaceutical composition at a mass percentage of 0.1% to 10%, preferably 0.5% to 6.5%.

In some embodiments, the composition further comprises at least one of a filler, a disintegrant, an adhesive and a lubricant.

In some embodiments, the composition also further comprises a glidant.

In some embodiments, the filler is selected from at least one of lactose, microcrystalline cellulose, mannitol, starch, sucrose, dextrin, dicalcium phosphate, calcium sulphate and calcium carbonate.

In some embodiments, the disintegrant is selected from at least one of croscarmellose sodium, crospovidone, sodium carboxymethyl starch and low-substituted hydroxypropyl cellulose.

In some embodiments, the adhesive is selected from at least one of hydroxypropyl cellulose, hypromellose, copovidone, povidone, ethyl cellulose and methyl cellulose.

In some embodiments, the lubricant is selected from at least one of magnesium stearate, calcium stearate, sodium stearyl fumarate and glyceryl behenate.

In some embodiments, the glidant is selected from at least one of silicon dioxide, talc and colloidal silicon dioxide.

In some embodiments, the disintegrant is present in the pharmaceutical composition at a mass percentage of 1% to 15%, preferably 2% to 5%.

In some embodiments, the adhesive is present in the pharmaceutical composition at a mass percentage of 1% to 20%, preferably 2% to 5%.

In some embodiments, the lubricant is present in the pharmaceutical composition at a mass percentage of 0.5% to 3%.

In some embodiments, the glidant is present in the pharmaceutical composition at a mass percentage of 0% to 5%, preferably 0% to 1%.

The present invention provides an oral pharmaceutical composition, comprising:
a) a compound of formula (I) or a pharmaceutically acceptable salt thereof, 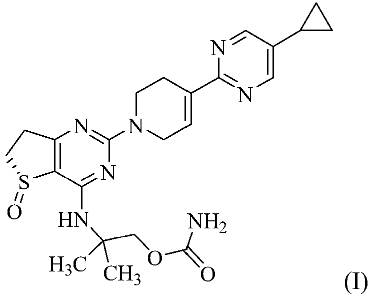 and
   a solubilizer and/or an acidity regulator.

In some embodiments, the solubilizer is selected from an ionic surfactant, a nonionic surfactant or a combination thereof, and the nonionic surfactant does not comprise polyethylene glycol-15-hydroxystearate.

In some embodiments, the ionic surfactant is selected from sulphonated castor oil, sodium dodecyl sulphate, sodium hexadecyl sulphate and sodium octadecyl sulphate.

In some embodiments, the nonionic surfactant is selected from Spans (Span 20, Span 40, Span 60, etc.), Tweens (Tween 60, Tween 80, etc.), Myrjs (Myrj 40, Myrj 45, etc.), Brijs (Brij 35 etc.) and poloxamers (poloxamer 188, poloxamer 407, etc.).

In some embodiments, the solubilizer is selected from at least one of sodium hexadecyl sulphate, sodium dodecyl sulphate, poloxamer 188, Tween 80 and Span 60.

In some embodiments, the acidity regulator is selected from at least one of citric acid, fumaric acid, citric acid, succinic acid, sorbic acid, oxalic acid and ascorbic acid.

In some embodiments, the acidity regulator is selected from at least one of citric acid, fumaric acid, succinic acid, sorbic acid, oxalic acid and ascorbic acid.

In some embodiments, the composition further comprises a filler, a disintegrant, an adhesive and a lubricant.

In some embodiments, the filler is selected from at least one of lactose, microcrystalline cellulose, mannitol, starch, sucrose, dextrin, dicalcium phosphate, calcium sulphate and calcium carbonate.

In some embodiments, the disintegrant is selected from at least one of croscarmellose sodium, crospovidone, sodium carboxymethyl starch and low-substituted hydroxypropyl cellulose.

In some embodiments, the adhesive is selected from at least one of hydroxypropyl cellulose, hypromellose, copovidone, povidone, ethyl cellulose and methyl cellulose.

In some embodiments, the lubricant is selected from at least one of magnesium stearate, calcium stearate, sodium stearyl fumarate and glyceryl behenate.

In some embodiments, the composition further comprises a glidant, and the glidant is selected from at least one of silicon dioxide, talc and colloidal silicon dioxide.

In some embodiments, the compound of formula (I) is present in the pharmaceutical composition at a mass percentage of 0.1% to 10%, preferably 0.5% to 7%.

In some embodiments, the compound of formula (I) is present in the pharmaceutical composition at a mass percentage of 0.1% to 10%, preferably 0.5% to 6.5%.

In some embodiments, the mass ratio of the solubilizer to the compound of formula (I) is 1 : 1 to 50 : 1, preferably 1 : 1 to 20 : 1.

In some embodiments, the acidity regulator is present in the pharmaceutical composition at a mass percentage of 1% to 20%.

In some embodiments, the disintegrant is present in the pharmaceutical composition at a mass percentage of 1% to 15%, preferably 2% to 5%.

In some embodiments, the adhesive is present in the pharmaceutical composition at a mass percentage of 1% to 20%, preferably 2% to 5%.

In some embodiments, the lubricant is present in the pharmaceutical composition at a mass percentage of 0.5% to 3%.

In some embodiments, the glidant is present in the pharmaceutical composition at a mass percentage of 0% to 5%, preferably 0% to 1%.

In some embodiments, the oral pharmaceutical composition is used to prepare a pharmaceutical preparation.

In some embodiments, the compound of formula (I) is present in a unit preparation thereof in an amount from 0.1 mg to 100 mg.

In some embodiments, the compound of formula (I) is present in a unit preparation thereof in an amount selected from 0.5 mg, 1 mg, 2 mg, 4 mg, 8 mg, or 10 mg, preferably 0.5 mg or 4 mg.

In some embodiments, the preparation is selected from a tablet, a capsule, a granule or a dispersible tablet.

In some embodiments, the preparation is a tablet, and a method for preparing the tablet comprises the steps of:
(1) mixing the compound of formula (I) with an auxiliary material uniformly; and
(2) compressing the mixture into the tablet.

In some embodiments, the method for preparing the tablet comprises the steps of:
(1) Weighing: weighing the active pharmaceutical ingredient (API)/auxiliary materials for granulation according to the formulation amount;
(2) Mixing: manually mixing some of the auxiliary materials with the API first, adding the remaining auxiliary materials in a granulator, mixing the blank auxiliary materials for 2 min, adding the API and stirring and mixing the mixture for 2 min, then starting shearing and mixing for 3 min;
(3) Granulation: starting stirring and shearing, adding an appropriate amount of wetting agent, carrying out granulation for 2 min, and removing the obtained granules;
(4) Wet sizing: carrying out wet sizing using a 20-mesh sieve;
(5) Drying: drying the granules in an oven at 60°C for 1-3 h and then removing same;
(6) Dry sizing: carrying out dry sizing using a 24-mesh sieve;
(7) Final blending: Weighing silicon dioxide and magnesium stearate, adding same to the dry granules at one time, and mixing the mixture uniformly;
(8) Tableting: adjusting the tableting parameters to obtain tablets with a target weight of about 60 mg or 70 mg, and maintaining tablet hardness greater than 25 N.

The optional main processes of the present invention include: wet granulation, dry granulation and direct powder compression.

Process characteristics of the present invention:
(1) A specific mixing process is used to mix the low-dose drug, thereby avoiding the problem of content uniformity associated with low-dose drugs;
(2) The API is pre-treated (micronized);
(3) The preparation process is as follows: micronization of API; mixing of API and auxiliary materials; wet granulation; drying; sizing; final blending; and tableting.

The oral pharmaceutical composition of the PDE4B inhibitor compound provided by the present invention has the advantages of a high bioavailability, good content uniformity, good stability, easy industrialization, etc.

Solubility test method of the present invention: purified water is used as the medium; media with different concentrations are prepared with various solubilizers; an excess amount of the API is added until visible precipitates are observed to the naked eye; the mixture is subjected to ultrasonication for 10 min before sample preparation; the concentration is determined by HPLC.

Dissolution profile determination method of the present invention: paddle method, 900 ml of dissolution medium, a rotation speed: 50 rpm, and temperature: 37.5°C. The dissolution rate is determined by HPLC. Sampling time point: 5/10/15/20/30/45/60 min.

### Detailed Description of Embodiments

The technical problems to be solved, technical solutions and beneficial effects of the present invention are described below in conjunction with examples. It should be understood that the specific examples described herein are merely used to explain the present invention, but are not intended to limit the present invention.

### Example 1

The solubility of the active pharmaceutical ingredient in solutions containing different concentrations of solubilizers was investigated. Sodium dodecyl sulphate (SDS), fumaric acid and citric acid were separately investigated.

| Solubility test results | | | | |
|---|---|---|---|---|
| Batch No. | Auxiliary material | Concentration | Solubility (mg/ml) | Fold increase |
| Y1 | SDS | 0.5% | 0.307 | 34.1 |
| Y2 | | 1.0% | 0.451 | 50.1 |
| Y3 | Fumaric acid | 5% | 0.217 | 24.1 |
| Y4 | | 10% | 0.246 | 27.3 |
| Y5 | Citric acid | 5% | 0.454 | 50.4 |
| Y6 | | 10% | 0.479 | 53.2 |
| Y7 | Purified water | / | 0.009 | 1.0 |

Fumaric acid was selected as the solubilizer to prepare samples, and the effect of the amount of fumaric acid on the preparation was investigated.

| | Formulation 1 | | | Formulation 2 | | |
|---|---|---|---|---|---|---|
| API/auxiliary materials | mg/tabl et | Percentage /% | 70 tablets/g | mg/tabl et | Percentage /% | 70 tablets/g |
| Compound of formula (I) | 4.00 | 5.71 | 0.280 | 4.00 | 5.71 | 0.280 |
| Mannitol | 47.60 | 68.00 | 3.332 | 44.10 | 63.00 | 3.087 |
| Microcrystalline cellulose | 10.00 | 14.29 | 0.700 | 10.00 | 14.29 | 0.700 |
| Fumaric acid | 3.50 | 5.00 | 0.245 | 7.00 | 10.00 | 0.490 |
| Crospovidone | 2.10 | 3.00 | 0.147 | 2.10 | 3.00 | 0.147 |
| Hydroxypropyl cellulose | 2.10 | 3.00 | 0.147 | 2.10 | 3.00 | 0.147 |
| Magnesium stearate | 0.70 | 1.00 | 0.049 | 0.70 | 1.00 | 0.049 |
| Total | 70.00 | 100.00 | 4.90 | 70.00 | 100.00 | 4.90 |

Sodium dodecyl sulphate was selected as the solubilizer to prepare samples, and the effect of the amount thereof on the preparation was investigated.

| | **Formulation 3** | | | **Formulation 4** | | | **Formulation 5 (without solubilizer)** | | |
|---|---|---|---|---|---|---|---|---|---|
| **API/auxiliar y materials** | **mg/ta blet** | **Percenta ge/%** | **70 tablets/ g** | **mg/ta blet** | **Percenta ge/%** | **70 tablets/ g** | **mg/ta blet** | **Percenta ge/%** | **70 table ts/g** |
| **API** | 4.00 | 5.71 | 0.280 | 4.00 | 5.71 | 0.280 | 4.00 | 6.67 | 0.280 |
| **Mannitol** | 41.10 | 58.71 | 2.877 | 31.10 | 44.43 | 2.177 | 39.8 | 66.33 | 2.786 |
| **Microcrystal line cellulose** | 10.00 | 14.29 | 0.700 | 10.00 | 14.29 | 0.700 | 10.8 | 18 | 0.756 |
| **Sodium dodecyl sulphate** | 10.00 | 14.29 | 0.700 | 20.00 | 28.57 | 1.400 | 0 | 0 | 0 |
| **Crospovidon e** | 2.10 | 3.00 | 0.147 | 2.10 | 3.00 | 0.147 | 3.0 | 5.00 | 0.21 |
| **Hydroxypro pyl cellulose** | 2.10 | 3.00 | 0.147 | 2.10 | 3.00 | 0.147 | 1.80 | 3.00 | 0.126 |
| **Magnesium stearate** | 0.70 | 1.00 | 0.049 | 0.70 | 1.00 | 0.049 | 0.60 | 1.00 | 0.042 |
| **Total** | 70.00 | 100.00 | 4.90 | 70.00 | 100.00 | 4.90 | 60.00 | 100.00 | 4.20 |

### Dissolution profile data for formulation 1 to formulation 5

| | Formulation 1 | | Formulation 2 | | Formulation 3 | | Formulation 4 | | Formulation 5 | |
|---|---|---|---|---|---|---|---|---|---|---|
| Ti me | Dissoluti on rate | RA D/% | Dissoluti on rate | RA D/% | Dissoluti on rate | RA D/% | Dissoluti on rate | RA D/% | Dissoluti on rate | RA D/% |
| 5 | 37.4 | 12.1 | 68.7 | -1.8 | 56.2 | -0.6 | 64.9 | 0.5 | 28.0 | -2.4 |
| 10 | 68.7 | -7.9 | 80.6 | 1.0 | 83.3 | -5.0 | 77.3 | 1.5 | 43.0 | -1.5 |
| 15 | 84.4 | -5.2 | 85.2 | 1.4 | 93.5 | -2.4 | 84.5 | 3.0 | 51.0 | -0.9 |
| 20 | 87.2 | -3.2 | 86.0 | 0.0 | 92.5 | -4.5 | 86.6 | 4.0 | 55.1 | -1.3 |
| 30 | 89.2 | -1.7 | 87.0 | 1.3 | 93.3 | -3.1 | 90.9 | 1.5 | 59.9 | -2.5 |
| 45 | 90.8 | -2.2 | 87.9 | 1.2 | 96.9 | -0.9 | 91.5 | 2.7 | 65.6 | 0.4 |
| 60 | 88.4 | -4.8 | 90.2 | 2.2 | 96.5 | -3.2 | 92.1 | 1.7 | 66.3 | -1.6 |

Conclusion: The solubilizer sodium dodecyl sulphate and the acidity regulator fumaric acid significantly improve the dissolution profile of the preparation, greatly increasing the dissolution rate thereof. The greater the amount of fumaric acid, the greater the dissolution rate thereof. Favourable dissolution rates are obtained when the amount ratio of sodium dodecyl sulphate to active pharmaceutical ingredient ranges from 2.5 : 1 to 5 : 1.

### Example 2 Pharmacokinetic study in dogs

Formulation information:

| Capsule | **Formulation 6** | | **Formulation 7** | | **Formulation 8** | |
|---|---|---|---|---|---|---|
| API/auxiliary materials | mg/tablet | Percentage/% | mg/tablet | Percentage/% | mg/tablet | Percentage/% |
| API | 4.00 | 4.00 | 4.00 | 4.00 | 4.00 | 4.00 |
| Mannitol 160C | 96.00 | 96.00 | 56.00 | 56.00 | 16.00 | 16.00 |
| SDS | 0.00 | 0.00 | 40.00 | 40.00 | 80.00 | 80.00 |
| Total | 100.00 | 100.00 | 100.00 | 100.00 | 100.00 | 100.00 |

| | **Formulation 9** | | **Formulation 10** | | **Formulation 11** | | **Formulation 12** | |
|---|---|---|---|---|---|---|---|---|
| API/auxiliar y materials | mg/tab let | Percentag e/% | mg/tab let | Percentag e/% | mg/tab let | Percentag e/% | mg/tab let | Percentag e/% |
| Compound of formula (I) | 4.00 | 5.95 | 4.00 | 5.60 | 4.00 | 5.60 | 4.00 | 3.59 |
| Mannitol 160C | 20.00 | 29.76 | 31.10 | 43.56 | 41.10 | 57.56 | 71.10 | 63.82 |
| Microcrysta line cellulose PH101 | 19.00 | 28.27 | 10.00 | 14.01 | 10.00 | 14.01 | 10.00 | 8.98 |
| Sodium dodecyl sulphate | 20.00 | 29.76 | 20.00 | 28.01 | 10.00 | 14.01 | 20.00 | 17.95 |
| Croscarmell ose sodium | 0.00 | 0.00 | 2.10 | 2.94 | 2.10 | 2.94 | 2.10 | 1.89 |
| Hypromello se E5 | 3.50 | 5.21 | 3.50 | 4.90 | 3.50 | 4.90 | 3.50 | 3.14 |
| Magnesium stearate | 0.70 | 1.04 | 0.70 | 0.98 | 0.70 | 0.98 | 0.70 | 0.63 |
| Total | 67.20 | 100.00 | 71.40 | 100.00 | 71.40 | 100.00 | 111.40 | 100.00 |

### Data of PK test in dogs

| **PO PK-4 mg/dog** | **Cₘₐₓ (ng/mL)** | **AUC_{0-48 h} (h·ng·mL⁻¹)** | **T_{1/2} (h)** | **Tₘₐₓ (h)** | **F%** |
|---|---|---|---|---|---|
| **Formulation 9** | 130+26.5 | 1075±131 | 9.57±0.79 | 2.67±1.2 | 49.8±4.0 |
| **Formulation 10** | 72.4±19.4 | 565±213 | 9.78±3.0 | 3.33±1.2 | 23.8±8.9 |
| **Formulation 11** | 146±10.7 | 873±190 | 7.28±1.2 | 1.67±0.58 | 41.1±8.9 |
| **Formulation 12** | 139±38.0 | 880±161 | 8.55±1.5 | 1.17±0.76 | 38.9±7.1 |
| **Formulation 6** | 89.8±22.1 | 645±109 | 7.86±2.7 | 1.33±0.58 | 26.2±4.0 |
| **Formulation 7** | 196±8.33 | 1074±76 | 7.32±6.2 | 1.67±0.58 | 42.5±0.28 |
| **Formulation 8** | 150±26.3 | 1021±171 | 16.2±8.1 | 3.33±1.2 | 39.7±6.6 |

| | | | | | |
|---|---|---|---|---|---|
| Formulation 9: API : sodium dodecyl sulphate = 1 : 5, disintegration time: 30 min Formulation 10: API : sodium dodecyl sulphate = 1 : 5, unmilled active pharmaceutical ingredient, disintegration time: 16-18 min Formulation 11: API : sodium dodecyl sulphate = 1 : 2.5, disintegration time: 16-17 min Formulation 12: API : sodium dodecyl sulphate = 1 : 5, disintegration time: less than 15 min Formulation 6: No sodium dodecyl sulphate, API filled directly into capsules Formulation 7: API : sodium dodecyl sulphate = 1 : 10 Formulation 8: API : sodium dodecyl sulphate = 1 : 20 Note: Unless otherwise specified, all active pharmaceutical ingredients are subjected to jet milling. | | | | | |

### Data analysis of PK test in dogs:

(1) The formulation containing un-micronized active pharmaceutical ingredient exhibited the lowest bioavailability in dogs;
(2) Formulation 12 with the shortest disintegration time did not exhibit the best bioavailability in dogs;
(3) Formulation 9 without a disintegrant had a longer disintegration time but exhibited the best bioavailability in dogs;
(4) Sodium dodecyl sulphate significantly improved the bioavailability in dogs; the bioavailability was effectively improved when the amount ratio of API : SDS was within the range of 1 : 2.5 to 1 : 20.

### Example 3: Preparation of compound of formula (I)

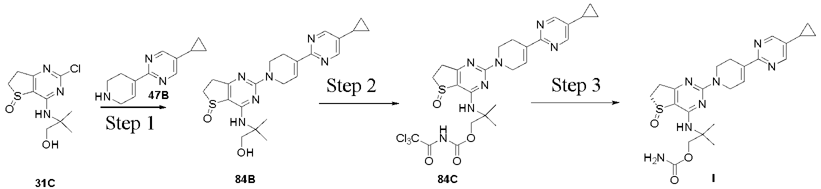

Step 1: Compound **31C** (1.0 g, 3.63 mmol) was dissolved in 1,4-dioxane (30 mL), and compound **47B** (880 mg, 4.36 mmol) and N,N-diisopropylethylamine (1.40 g, 10.86 mmol) were added. The mixture was stirred at 90°C overnight under nitrogen atmosphere. After the reaction was completed, the reaction solution was concentrated and subjected to silica gel column chromatography (dichloromethane/methanol (v/v) = 95/5) to obtain compound **84B** (1.6 g, 94%).
LC-MS (ESI): m/z = 441.6 [M+H]⁺

Step 2: Compound **84B** (1.6 g, 3.63 mmol) was dissolved in dichloromethane (5 mL), and trichloroacetyl isocyanate (820 mg, 4.36 mmol) was added in an ice bath. The mixture was stirred in the ice bath for one hour. The reaction solution was concentrated to obtain compound **84C** (2.28 g, 100%).

Step 3: Compound **84C** (2.28 g, 3.63 mmol) was dissolved in methanol (20 mL), and potassium carbonate (1.51 g, 10.89 mmol) and water (20 mL) were added in an ice bath. The mixture was stirred at room temperature for 2.5 hours. The reaction solution was diluted with water and extracted with dichloromethane. The organic phases were combined, dried, filtered, concentrated, and subjected to SFC chiral resolution to obtain compound (I) (1.3 g, 74%).

Preparation method: instrument: Waters 150 Prep-SFC E, column: Chiralcel OX column (250 mm × 30 mm, 10 µm), mobile phase: (phase A: CO₂, phase B: EtOH (0.1%NH₃•H₂O)); gradient: 50% mobile phase B, isocratic elution; flow rate: 100 mL/min, back pressure: 100 bar, column temperature: 25°C, wavelength: 220 nm, cycle time: 7.0 min, sample preparation: sample concentration 10 mg/ml, acetonitrile solution injection: 8 ml each time. Retention time: 1.499 min. After separation, the fraction was dried in a water bath at 35°C using a rotary evaporator to obtain a product. The solvent was dried by a lyophiliser at -80°C to obtain the final product, the compound of formula (I).

¹H NMR (400 MHz, DMSO-*d₆*) δ 8.54 (s, 2H), 7.19 (d, 1H), 6.70 - 6.40 (m, 3H), 4.46 (s, 2H), 4.30 (s, 2H), 4.00 (t, 2H), 3.48 - 3.38 (m, 1H), 3.28 - 3.23 (m, 1H), 3.01 - 2.92 (m, 1H), 2.92 - 2.82 (m, 1H), 2.67 (s, 2H), 1.98 - 1.88 (m, 1H), 1.46 (d, 6H), 1.10 - 0.96 (m, 2H), 0.91 - 0.77 (m, 2H).
LC-MS (ESI): m/z = 484.2 [M+H]⁺

### Preparation of compound 31C:

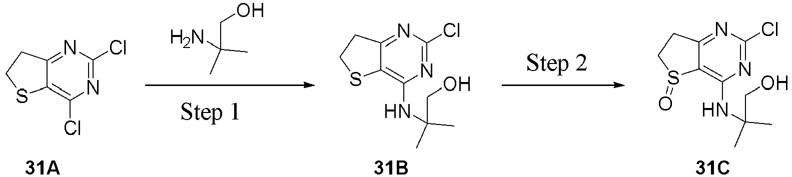

Step 1: Compound **31A** (2.0 g, 9.64 mmol) was dissolved in acetonitrile (20 mL), and 2-amino-2-methylpropan-1-ol (950.0 mg, 10.64 mmol) and triethylamine (2.93 g, 28.92 mmol) were then added. The mixture was heated to 70°C and reacted for 14 h. The reaction solution was concentrated and subjected to column chromatography (petroleum ether/ethyl acetate = 1/1) to obtain compound **31B** (600 mg, 24%).
LC-MS (ESI): m/z = 260.1 [M+1]⁺

Step 2: Compound **31B** (600.0 mg, 2.31 mmol) was dissolved in dichloromethane (10 mL), and m-chloroperoxybenzoic acid (600.0 mg, 3.49 mmol) was added. The mixture was stirred for 16 hours. The reaction solution was quenched with a saturated sodium thiosulphate aqueous solution. The aqueous phase was extracted with dichloromethane, dried, concentrated, and subjected to column chromatography (dichloromethane/methanol = 10/1) to obtain compound **31C** (632.0 mg, 99%).
LC-MS (ESI): m/z = 276.1 [M+H]⁺

Step 3: Compound **31C** (632.0 mg, 2.29 mmol) was dissolved in 1,4-dioxane (10.0 mL), and compound **9D** (540.0 mg, 2.75 mmol) and N,N-diisopropylethylamine (890 mg, 6.87 mmol) were then added. The mixture was heated to 90°C and reacted for 12 h. The reaction solution was cooled to room temperature, concentrated, and subjected to column chromatography (dichloromethane/methanol = 10/1) to obtain compound **31** (440.0 mg, 44%).
LC-MS (ESI): m/z = 435.1 [M+H]⁺

¹H NMR (400 MHz, DMSO-*d₆*) δ 8.90 (s, 2H), 7.38 - 7.16 (m, 1H), 6.23 (s, 1H), 5.15 (t, 1H), 4.49 (s, 2H), 4.00 (t, 2H), 3.51 (d, 2H), 3.48 - 3.33 (m, 2H), 3.01 - 2.91 (m, 1H), 2.91 - 2.82 (m, 1H), 2.67 (s, 2H), 1.43 (s, 6H).

### Biological test:

### 1. Effect of compounds on PDE4B2 activity

The effect of compounds on PDE4B2 activity was detected using a fluorescence polarization kit (BPS Bioscience, Catalogue #60343). According to the instructions of the kit, FAM-Cyclic-3',5'-AMP at a final concentration of 0.1 µM, 1 ng/well of PDE4B2 (for a negative control, a PDE buffer was added), and serially diluted compounds (for a positive control well, a PDE buffer containing 10% of DMSO was added) were added to each well, fully mixed and then reacted at room temperature for 1 h. The Binding Agent was diluted with the Binding Agent Diluent (cAMP) at a ratio of 1 : 100 for later use, and the Binding Agent dilution was taken and added to the test plate at 50 µl/well and incubated at room temperature with slow shaking for 20 minutes. After the incubation was complete, Envision was used for FP detection, with Excitation at 480 nm and Emission at 535 nm. FP was usually represented by mP value. $mP = \left(\frac{{I}_{II} - G \left({I}_{⊥}\right)}{{I}_{II} + G \left({I}_{⊥}\right)}\right) \times 1000$
III (S535): fluorescence intensity in parallel direction
I_{┴} (P535): fluorescence intensity in perpendicular direction
G: G factor = 1
Calculation of inhibition rate (% Inhibition): 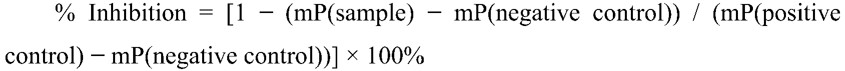 % Inhibition = [1 - (mP(sample) - mP(negative control)) / (mP(positive control) - mP(negative control))] × 100%
mP(sample): mP of the test compound in a reaction well
mP(negative control): mP in the negative control well
mP(positive control): mP in the positive control well
On the basis of the calculated inhibition rate at each concentration, the IC₅₀ value of each compound was calculated using GraphPad Prism 8 software.

The compounds of the present invention had an IC₅₀ value < 300 nM against PDE4B2; preferably, some compounds had an IC₅₀ value < 100 nM; more preferably, some compounds had an IC₅₀ value < 50 nM; and further preferably, some compounds had an IC₅₀ value < 10 nM.

The compounds of the present invention had an IC₅₀ value of less than 300 nM against PDE4B2, preferably less than 100 nM, more preferably less than 50 nM, and further preferably less than 10 nM. The IC₅₀ values of some specific compounds are as shown in the table below, wherein A < 10 nM, 10 nM ≤ B < 50 nM, and 50 nM ≤ C < 100 nM.

### PDE4B2 activity

| Compound | PDE4B2 IC₅₀ (nM) |
|---|---|
| Compound of formula (I) | A |

### 2. Effect of compounds on PDE4D2 activity

The effect of compounds on PDE4D2 activity was detected using a fluorescence polarization kit (BPS Bioscience, Catalogue #60345). During the reaction, 12.5 µL of 0.272 ng/well of an enzyme (final concentration: 0.068 ng/well) and 12.5 µL of the compound diluted in a gradient manner (DMSO concentration: 4%) were first pre-incubated at room temperature for 15 minutes, the same volume of the enzyme at the same concentration and 12.5 µL of PDE buffer containing 4% DMSO were added to the positive control well, and 25 µL of PDE buffer containing 2% DMSO was added to the negative control well. After completion, 25 µL of 0.2 µM FAM-Cyclic-3',5'-AMP (final concentration: 0.1 µM) was added to each well, fully mixed, and then incubated at room temperature with slow shaking for 30 minutes. The Binding Agent was diluted with the Binding Agent Diluent (cAMP) at a ratio of 1 :100 for later use, and the Binding Agent dilution was taken and added to all the wells of the test plate at 100 µl/well and incubated at room temperature with slow shaking for 1 hour. After the incubation was complete, BMG LRBTECH microplate reader was used for FP detection, with Excitation at 485 nm and Emission at 520 nm. FP was usually represented by mP value. $mP = \left(\frac{{I}_{II} - G \left({I}_{⊥}\right)}{{I}_{II} + G \left({I}_{⊥}\right)}\right) \times 1000$
III (S520): fluorescence intensity in parallel direction
I_{┴} (P520): fluorescence intensity in perpendicular direction
G: G factor = 1
Calculation of inhibition rate (% Inhibition): 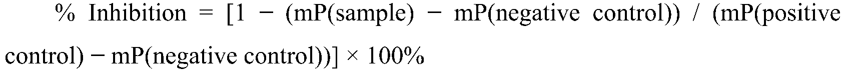 % Inhibition = [1 - (mP(sample) - mP(negative control)) / (mP(positive control) - mP(negative control))] × 100%
mP(sample): mP of the test compound in a reaction well
mP(negative control): mP in the negative control well
mP(positive control): mP in the positive control well
On the basis of the calculated inhibition rate at each concentration, the IC₅₀ value of each compound was calculated using GraphPad Prism 8 software.

The IC₅₀ values of some specific compounds of the present invention are as shown in the table below, wherein A < 10 nM, 10 nM ≤ B < 50 nM, and 50 nM ≤ C < 100 nM.

### PDE4D2 activity

| Compound | PDE4D2 IC₅₀ (nM) |
|---|---|
| Compound of formula (I) | A |

### 3. Detection of inhibitory activity of compounds on the release of tumour necrosis factor-α (TNF-α) from human peripheral blood mononuclear cells induced by lipopolysaccharide (LPS) in vitro

Normal human peripheral anticoagulated blood (citrate anticoagulation) was collected, and hPBMCs were prepared using Ficoll-Paque PLUS (Cytiva, Cat# 17144002, density 1.077 g/mL). The concentration of hPBMC cells was adjusted to 0.25 × 10⁶ cells/mL with RPMI1640 medium and inoculated into a 96-well plate at 50,000 cells/well. Subsequently, different concentrations of test compounds were added and pre-incubated for 1 h (final DMSO concentration: 0.1%; positive and negative control wells received an equal volume of RPMI1640 containing 0.1% DMSO). After the pre-incubation was completed, 100 ng/mL LPS (SIGMA, Cat. No. L2630) was added to the compound and positive control wells, while an equal volume of RPMI1640 medium containing 0.1% DMSO was added to a negative control well. The plate was incubated in an incubator at 37°C with 5% CO₂ for 4 h. The cell supernatant was collected, and the content of TNF-α in the supernatant sample was detected by the human TNF-α Elisa quantitative test kit (Sino Biological, Cat# KIT10602). The IC₅₀ value was calculated using GraphPad Prism software. The IC₅₀ values of some specific compounds of the present invention are as shown in the table below, wherein A < 10 nM, 10 nM ≤ B < 50 nM, and 50 nM ≤ C < 100 nM.

Inhibitory activity of compounds on TNF-α induced by LPS *in vitro*

| Test compound | **Re** IC₅₀ (nM) | **Max inhibition** |
|---|---|---|
| | | **(%)** |
| **Compound of formula (I)** | A | 68.04 |

| | | |
|---|---|---|
| Re IC₅₀: relative IC₅₀. | | |

### 4 Pharmacokinetic test in rats

**4.1 Experimental animals:** Male SD rats, about 220 g, 6-8 weeks old, six rats/compound, purchased from Chengdu Dossy Experimental Animals Co., Ltd.

**4.2 Experimental design:** On the day of the experiment, 6 SD rats were randomly grouped according to body weight. The animals were fasted with free access to water for 12-14 h one day before the administration, and were fed 4 h after the administration.

### Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound of formula (I) | 2.5 | 0.5 | 5 | Plasma | Intravenous administration |
| G2 | 3 | | 10 | 1 | 10 | Plasma | Intragastric administration |

| | | | | | | | |
|---|---|---|---|---|---|---|---|
| Note: Vehicle for intravenous administration: 10% DMA+10% Solutol+80% Saline; vehicle for intragastric administration: 0.5% MC (DMA: dimethylacetamide; Solutol: polyethylene glycol-15-hydroxystearate; Saline: physiological saline; MC: methylcellulose) | | | | | | | |

Before and after administration, 0.15 ml of blood was taken from the orbit under isoflurane anaesthesia and placed in an EDTAK2 centrifuge tube. The tube was centrifuged at 5000 rpm at 4°C for 10 min, and plasma was collected. The blood collection time points for both the intravenous administration group and intragastric administration group were: 0, 5 min, 15 min, 30 min, 1 h, 2 h, 4 h, 6 h, 8 h and 24 h. Before analysis and detection, all samples were stored at -80°C, and the samples were quantitatively analysed by LC-MS/MS.

Pharmacokinetic parameters of test compound in plasma of rats

| Test compound | Mode of administration | CL (mL/min/kg) | Vdₛₛ (L/kg) | AUC₀₋ₜ (hr*ng/mL) | F (%) |
|---|---|---|---|---|---|
| Compound of formula (I) | i.v. (2.5 mg/kg) | 2.54±0.32 | 0.324±0.012 | 16531±1991 | - |
| | i.g. (10 mg/kg) | - | - | 52179±21323 | 78.9±32 |

| | | | | | |
|---|---|---|---|---|---|
| -: not applicable. | | | | | |

Conclusion: The compound as shown in formula (I) of the present invention exhibits excellent pharmacokinetic properties in the PK test in mice.

## Claims

1. An oral pharmaceutical composition, **characterised by** comprising:
an active ingredient, wherein the active ingredient is a compound of formula (I) or a pharmaceutically acceptable salt thereof, 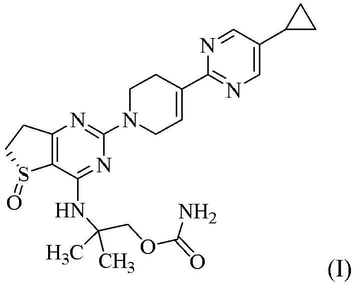
wherein the active ingredient is present in the pharmaceutical composition at a mass percentage of 0.1% to 10%, preferably 0.5% to 6.5%.

2. The composition according to claim 1, **characterised in that** the composition further comprises at least one of a filler, a disintegrant, an adhesive and a lubricant; optionally, the composition also further comprises a glidant.

3. The composition according to claim 2, **characterised in that** the filler is selected from at least one of lactose, microcrystalline cellulose, mannitol, starch, sucrose, dextrin, dicalcium phosphate, calcium sulphate and calcium carbonate;
the disintegrant is selected from at least one of croscarmellose sodium, crospovidone, sodium carboxymethyl starch and low-substituted hydroxypropyl cellulose;
the adhesive is selected from at least one of hydroxypropyl cellulose, hypromellose, copovidone, povidone, ethyl cellulose and methyl cellulose;
the lubricant is selected from at least one of magnesium stearate, calcium stearate, sodium stearyl fumarate and glyceryl behenate;
the glidant is selected from at least one of silicon dioxide, talc and colloidal silicon dioxide.

4. The composition according to any one of claims 2-3, **characterised in that** the disintegrant is present in the pharmaceutical composition at a mass percentage of 1% to 15%, preferably 2% to 5%;
the adhesive is present in the pharmaceutical composition at a mass percentage of 1% to 20%, preferably 2% to 5%;
the lubricant is present in the pharmaceutical composition at a mass percentage of 0.5% to 3%;
the glidant is present in the pharmaceutical composition at a mass percentage of 0% to 5%, preferably 0% to 1%.

5. An oral pharmaceutical composition, **characterised by** comprising:
a) a compound of formula (I) or a pharmaceutically acceptable salt thereof, 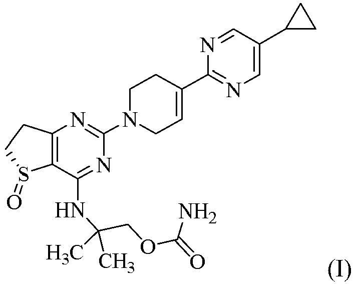 and
b) a solubilizer and/or an acidity regulator.

6. The composition according to claim 5, **characterised in that** the solubilizer is selected from an ionic surfactant, a nonionic surfactant or a combination thereof, and the nonionic surfactant does not comprise polyethylene glycol-15-hydroxystearate; optionally, the ionic surfactant is selected from sulphonated castor oil, sodium dodecyl sulphate, sodium hexadecyl sulphate and sodium octadecyl sulphate; the nonionic surfactant is selected from Spans, Tweens, Myrjs, Brijs and poloxamers; further, the solubilizer is selected from at least one of sodium hexadecyl sulphate, sodium dodecyl sulphate, poloxamer 188, Tween 80 and Span 60;
optionally, the acidity regulator is selected from at least one of citric acid, fumaric acid, citric acid, succinic acid, sorbic acid, oxalic acid and ascorbic acid.

7. The composition according to any one of claims 5-6, **characterised in that** the composition further comprises at least one of a filler, a disintegrant, an adhesive and a lubricant; optionally, the composition also further comprises a glidant.

8. The composition according to claim 7, **characterised in that** the filler is selected from at least one of lactose, microcrystalline cellulose, mannitol, starch, sucrose, dextrin, dicalcium phosphate, calcium sulphate and calcium carbonate;
the disintegrant is selected from at least one of croscarmellose sodium, crospovidone, sodium carboxymethyl starch and low-substituted hydroxypropyl cellulose;
the adhesive is selected from at least one of hydroxypropyl cellulose, hypromellose, copovidone, povidone, ethyl cellulose and methyl cellulose;
the lubricant is selected from at least one of magnesium stearate, calcium stearate, sodium stearyl fumarate and glyceryl behenate;
the glidant is selected from at least one of silicon dioxide, talc and colloidal silicon dioxide.

9. The composition according to any one of claims 5-8, **characterised in that** the compound of formula (I) is present in the pharmaceutical composition at a mass percentage of 0.1% to 10%, preferably 0.5% to 6.5%;
the mass ratio of the solubilizer to the compound of formula (I) is 1 : 1 to 50 : 1, preferably 1 : 1 to 20 : 1;
the acidity regulator is present in the pharmaceutical composition at a mass percentage of 1% to 20%;
the disintegrant is present in the pharmaceutical composition at a mass percentage of 1% to 15%, preferably 2% to 5%;
the adhesive is present in the pharmaceutical composition at a mass percentage of 1% to 20%, preferably 2% to 5%;
the lubricant is present in the pharmaceutical composition at a mass percentage of 0.5% to 3%;
the glidant is present in the pharmaceutical composition at a mass percentage of 0% to 5%, preferably 0% to 1%.

10. A pharmaceutical preparation prepared from the oral pharmaceutical composition according to any one of claims 1-9, **characterised in that** optionally, the compound of formula (I) is present in a unit preparation thereof in an amount from 0.1 mg to 100 mg, for example, in an amount selected from 0.5 mg, 1 mg, 2 mg, 4 mg, 8 mg or 10 mg.
